# EUROPEAN PATENT SPECIFICATION

(11) **EP 1 091 768 B1**
(45) Date of publication and mention of the grant of the patent: **05.11.2003**
(21) Application number: 99926662.0
(22) Date of filing: 23.06.1999
(51) Int. Cl.: A61L 9/14, A61L 9/22

(54) **TREATMENT OF AIRBORNE MICROORGANISMS**
BEHANDLUNG VON IN DER LUFT ENTHALTENEN MIKROORGANISMEN
TRAITEMENT DES MICRO-ORGANISMES EN SUSPENSION DANS L'AIR

(30) Priority: 02.07.1998 GB 9814377
(43) Date of publication of application: 18.04.2001
(73) Proprietor: UNIVERSITY OF SOUTHAMPTON, Southampton, Hampshire SO17 1BJ (GB); Reckitt Benckiser (UK) LIMITED, Slough, Berkshire SL1 3UH (GB)
(72) Inventor: HUGHES, John Farrell, Bartley, Southampton SO40 2JL (GB); FOX, Rodney Thomas, Cottingham, Hull HU16 4AS (GB); HARRISON, Mark Neale, Tutbury, Burton-on-Trent DE13 9HZ (GB); WHITMORE, Lindsey Faye, Colden Coommon, Winchester SO21 1UQ (GB); HARPER, Duncan Roger, Hull HU3 2TA (GB); JERRIM, Karen Louise, Eastleight, Hants SO50 7FN (GB); KNAPP, Jennifer, Jane, Southampton SO17 2FZ (GB)
(74) Representative: Dickson, Elizabeth Anne
(86) International application number: GB9901979
(87) International publication number: WO00001423

(56) References cited:
- EP-A- 0 673 656
- US-A- 5 403 587
- DATABASE WPI ON EPOQUE, week 9726, London: Derwent Publications Ltd., AN 97-287744, Class D22, XP002900667 & RU 2068706 A (UNIV TARTUS), abstract.

## Description

The present invention relates to the treatment of airborne microorganisms and viruses.

Disinfectants and sanitising compositions based on essential oils are known, for example from US Patent No. 5403587. This Patent is concerned with antimicrobial compositions for use in sanitising, disinfecting and/or cleaning hard surfaces such as countertops, tiles, porcelain products such as sinks and toilets, floors, windows, eating utensils, glassware, dishes and dental and surgical instruments. The compositions comprise:-
a) an anti-microbially effective amount of an essential oil capable of being dissolved or. dispersed in a water carrier and exhibiting antimicrobial properties when incorporated in a water carrier;
b) a solubilising or dispersing amount of a solubilising or dispersing agent sufficient. to form an aqueous solution or dispersion of the essential oil in a water carrier; and
c) sufficient water to make 100 wt percentage.

Essential oils stated to be of use in the invention as disclosed in US Patent No. 5403587 include oils obtained from thyme, lemon grass, lemons, oranges, anise, clove, roses, lavender, citronella, eucalyptus, peppermint, camphor, sandalwood and cedar.

The compositions of US Patent No. 5403587 are stated to be capable of being formulated with conventional propellants for dispensing as aerosols from conventional pressurised containers. Propellants which can be used include isobutane, n-butane, propane, dimethyl ether and blends thereof, as well as chlorofluorohydrocarbons, fluorohydrocarbons and mixtures thereof.

It is known to be difficult to treat airborne microorganisms. In general, it is not easy to eliminate them completely from a particular space such as that defined by a room. Furthermore, any aggressive form of treatment, such as the use of a spray of a composition which is toxic to the microorganisms is likely to be a health hazard to human or animals within the space being treated.

Bacteria, viruses and fungal spores can be considered to be particulate in nature when they are airborne, particularly since they are often attached to or associated with dust particles. In the case of the use of an aerosol spray device, a liquid composition containing a disinfectant is sprayed in the form of tiny droplets in the space which is to be disinfected. However, a low collision rate between the liquid droplets and the microorganisms in the air results in an ineffective killing of the microorganisms. The practical consequence of such inefficiency is that the disinfectant composition would need to be used in a high amount, thereby incurring a health risk. There are other possible side effects including, in the use of a perfumed composition, a resultant strong perfume smell because of the need to use a considerable amount of disinfectant composition and/or a limited fragrance choice.

RU-A-2068706 discloses a method for disinfecting and cleaning air involving spraying with a bactercidal preparation, electrical charging of the preparation and the air, and removal of the particles by electrostatic spraying. The sprayed preparation is subjected to ultraviolet irradiation at an energy density of 700 to 1500 Joule/cu.m. over 10 to 20 minutes.

An aerosol spray type device would be of improved efficacy if the aerosol spray droplets had a higher collision rate with the microorganisms. We have now developed an improved method of disinfecting or sanitising a space.

According to the present invention there is provided a method of disinfecting or sanitising a space occupied by airborne microorganisms and/or viruses, which method comprises directing into the space liquid droplets from a spray device containing a disinfecting or sanitising composition, a unipolar charge being imparted to the said liquid droplets solely by double layer charging during the spraying of the liquid droplets from the spray device, the unipolar charge being at a level such that the said droplets have a charge to mass ratio of at least +/- 1 x 10⁻⁴ C/kg.

The disinfecting or sanitizing composition which is sprayed in the method of the present invention contains at least one anti-microbial agent. Examples of such anti-microbial agents are essential oils such as thyme, lemon grass, lemon, orange, grapefruit, yeast, oregano, anise, clove, cinnamaldehyde, cinnamon, carvacrol, rose, lavender, citronella, eucalyptus, peppermint, camphor, sandalwood, Siberian pine needle, pine sylvester, tea tree, juniper berry, litsea, rosewood, patchouli, vetyver, cedarwood and mixtures thereof. Other anti-microbial agents which may be used in the present invention include bactericides, for example quarternary ammonium compounds such as alkyl dimethyl benzyl ammonium saccharinate and benzalkonium chloride, or fungicides such as clotrimazole, miconazole nitrate, organotin compounds, organic acids, halogenated phenols, quaternary ammonium compounds, 8-hydroxy quinoline, diamidines, organic mercury derivatives and the parabens.

It is preferred that the unipolar charge which is imparted to the liquid droplets is generated solely by the interaction between the liquid within the spray device and the spray device itself as the liquid is sprayed therefrom. In particular, it is preferred that the manner in which a unipolar charge is imparted to the liquid droplets does not rely even partly upon the connection of the device to any external charge inducing device, such as a source of relatively high voltage or any internal charge inducing device, such as a battery. With such an arrangement, the spray device is entirely self-contained making it suitable for use both in industrial, institutional and domestic environments.

Preferably, the spray device is a domestic pressure-spraying device devoid of any electrical circuitry but which is capable of being hand held. Typically such a device has a capacity in the range of from 10ml to 2000ml and can be actuated by hand, or by an automatic actuating mechanism. A particularly preferred domestic device is a hand-held aerosol can.

Preferably, therefore the droplet charge to mass ratio of at least +/- 1 x 10⁻⁴ C/kg is imparted to the droplets as a result of the use of an aerosol spray device with at least one of the features of the material of the actuator, the size and shape of the orifice of the actuator, the diameter of the dip tube, the characteristics of the valve and the formulation of the disinfecting or sanitising composition contained within the aerosol spray device being chosen in order to achieve the said droplet charge to mass ratio by double layer charging imparting the unipolar charge to the droplets during the actual spraying of the liquid droplets from the orifice of the aerosol spray device.

As a result of the method of the present invention, airborne microorganisms and/or viruses can be eliminated with considerable efficiency as compared to known spraying methods. In particular, much less disinfectant or sanitising agent is required than has previously been the case.

This result is achieved because of the unipolar charge imparted to the liquid droplets of the aerosol spray. This charge has two effects. The individual droplets are attracted to the microorganisms and/or viruses, including microorganisms attached to dust particles. Since all of the droplets carry the same polarity charge, they are repelled one from another. Accordingly, there is little or no coalescence of the droplets and, in contrast, they tend to spread out to a great extent as compared to uncharged droplets. In addition, if the repulsive forces from the charge within the droplets is greater than the surface tension force of the droplets, the charged droplets are caused to fragment into a plurality of smaller charged droplets (exceeding the Rayleigh limit). This process continues until either the two opposing forces are equalised or the droplet has fully evaporated.

Airborne microorganisms, including those attached to dust particles, are normally electrically isolated from their surroundings and will typically be at a potential which is the same as that of their surroundings. In this situation, an isolated microorganism within a cloud of electrically charged liquid droplets thus is likely to cause a distortion in the configuration of the electrical field generated by the droplets so that the attraction of the droplets onto the microorganism will be improved. In effect, the microorganism is targeted by a liquid droplet. This improvement in the interaction between the charged droplets and the microorganisms is due to the combined effect of the additional diffusion forces generated within the charged cloud of droplets by the electric field, leading to a modification of the trajectory of each droplet so that it is directed towards a microorganism.

In general, the liquid composition which is sprayed into the air using the aerosol spray device is preferably a water and hydrocarbon mixture, or emulsion, or a liquid which is converted into an emulsion by shaking the spraying device before use, or during the spraying process.

Whilst all liquid aerosols are known to carry a net negative or positive charge as a result of double layer charging, or the fragmentation of liquid droplets, the charge imparted to droplets of liquid sprayed from standard devices is only of the order of +/- 1 x 10⁻⁸ to 10⁻⁵ C/kg.

The invention relies on combining various characteristics of the design of an aerosol spray device so as to increase the charging of the liquid as it is sprayed from the aerosol spray device.

A typical aerosol spray device comprises:
1. An aerosol can containing the composition to be sprayed from the device and a liquid or gaseous propellant;
2. A dip tube extending into the can, the upper end of the dip tube being connected to a valve;
3. An actuator situated above the valve which is capable of being depressed in order to operate the valve; and
4. An insert provided in the actuator comprising an orifice from which the composition is sprayed.

A preferred aerosol spray device for use in the present invention is described in WO 97/12227.

It is possible to impart higher charges to the liquid droplets by choosing aspects of the aerosol device including the material, shape and dimensions of the actuator, the actuator insert, the valve and the dip tube and the characteristics of the liquid which is to be sprayed, so that the required level of charge is generated as the liquid is dispersed as droplets.

A number of characteristics of the aerosol system increase double layer charging and charge exchange between the liquid formulation and the surfaces of the aerosol system. Such increase are brought about by factors which may increase the turbulence of the flow through the system, and increase the frequency and velocity of contact between the liquid and the internal surfaces of the container and valve and actuator system.

By way of example, characteristics of the actuator can be optimised to increase the charge levels on the liquid sprayed from the container. A small orifice in the actuator insert, of a size of 0.45mm or less, increases the charge levels of the liquid sprayed through the actuator. The choice of material for the actuator can also increase the charge levels on the liquid sprayed from the device with material such as nylon, polyester, acetal, PVC and polypropylene tending to increase the charge levels. The geometry of the orifice in the insert can be optimised to increase the charge levels on the liquid as it is sprayed through the actuator. Inserts which promote the mechanical break-up of the liquid give better charging.

The actuator insert of the spray device may be formed from a conducting, insulating semi-conducting or static-dissipative material.

The characteristics of the dip tube can be optimised to increase levels in the liquid sprayed from the container. A narrow dip tube, of for example about 1.27mm internal diameter, increases the charge levels on the liquid, and the dip tube material can also be changed to increase charge.

Valve characteristics can be selected which increase the charge to mass ratio of the liquid product as it is sprayed from the container. A small tailpiece orifice in the housing, of about 0.65mm, increases product charge to mass ratio during spraying. A reduced number of holes in the stem, for example 2 x 0.50mm, also increases product charge during spray. The presence of a vapour phase tap helps to maximise the charge levels, a large orifice vapour phase tap of, for example, about 0.50mm to 1.0mm generally giving higher charge levels.

Changes in the product formulation can also affect charging levels. A formulation containing a mixture of hydrocarbon and water, or an emulsion of an immiscible hydrocarbon and water, will carry a higher charge to mass ratio when sprayed from the aerosol device than either a water alone or hydrocarbon alone formulation.

It is preferred that the microorganism treatment composition for use in the present invention comprises an oil phase, an aqueous phase, a surfactant, an antibacterial or anti-viral agent and a propellant.

Preferably the oil phase includes a C₉ - C₁₂ hydrocarbon which is preferably present in the composition in the amount of from 2 to 10% w/w.

Preferably the surfactant is glyceryl oleate or a polyglycerol oleate, preferably present in the composition in an amount of from 0.1 to 1.0% w/w.

Preferably the propellant is liquified petroleum gas (LPG) which is preferably butane, optionally in admixture with propane. The propellant may be present in an amount of from 10 to 90% w/w depending upon whether the composition is intended for spraying as a "wet" or as a "dry" composition. For a "wet" composition, the propellant is preferably present in an amount of from 20 to 50% w/w, more preferably in an amount of from 30 to 40% w/w.

The liquid droplets sprayed from the aerosol spray device will generally have diameters in the range of from 5 to 100 micrometres, with a peak of droplets of about 40 micrometres. The liquid which is sprayed from the aerosol spray device may contain a predetermined amount of a particulate material, for example, fumed silica, or a predetermined amount of a volatile solid material, such as menthol or naphthalene.

The method of the present invention, in addition to killing microorganisms, also accelerates the natural process of precipitation of airborne particles by indirect charging of the particles, thereby enabling the air quality to be improved quickly and conveniently.

A can for a typical aerosol spray device is formed of aluminium or lacquered or unlacquered tin plate or the like. The actuator insert may be formed of, for instance, acetal resin. The valve stem lateral opening may typically be in the form of two apertures of diameters 0.51mm.

The present invention will now be described, by way of example only, with reference to the accompanying drawings, in which:-
Figure 1 is a diagrammatic cross section through an aerosol spraying apparatus in accordance with the invention;
Figure 2 is a diagrammatic cross section through the valve assembly of the apparatus of Figure 1;
Figure 3 is a cross section through the actuator insert of the assembly shown in Figure 2;
Figure 4 shows the configuration of the bore of the spraying head shown in Figure 3 when viewed in the direction A; and
Figure 5 shows the configuration of the swirl chamber of the spraying head shown in Figure 3 when viewed in the direction B.

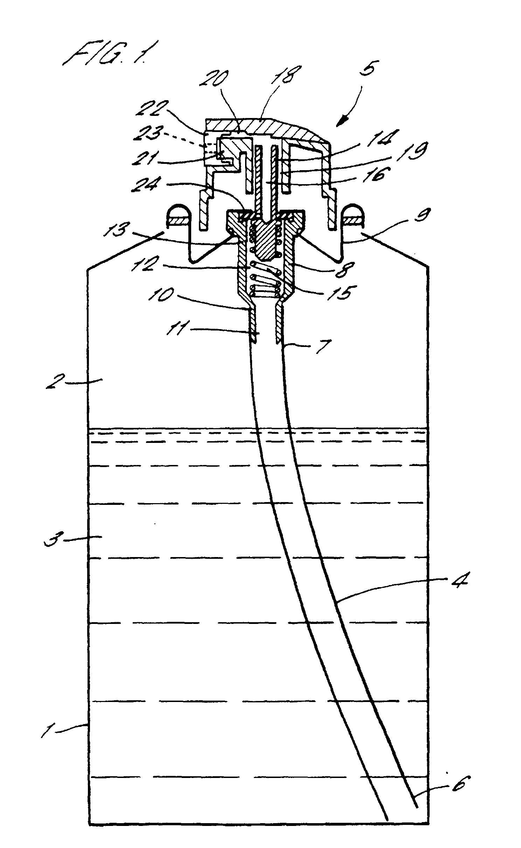

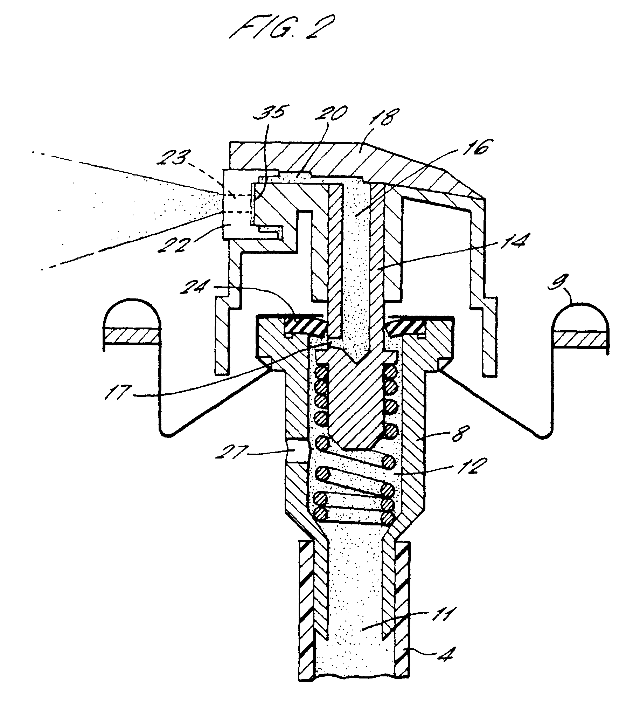

Referring to Figures 1 and 2, an aerosol spray device in accordance with the invention is shown. It comprises a can 1, formed of aluminium or lacquered or unlacquered tin plate or the like in conventional manner, defining a reservoir 2 for a liquid 3 having a conductivity such that droplets of the liquid can carry an appropriate electrostatic charge. Also located in the can is a gas under pressure which is capable of forcing the liquid 3 out of the can 1 via a conduit system comprising a dip tube 4 and a valve and actuator assembly 5. The dip tube 4 includes one end 6 which terminates at a bottom peripheral part of the can 1 and another end 7 which is connected to a tailpiece 8 of the valve assembly. The tailpiece 8 is secured by a mounting assembly 9 fitted in an opening in the top of the can and includes a lower portion 10 defining a tailpiece orifice 11 to which end 7 of the dip tube 4 is connected. The tailpiece includes a bore 12 of relatively narrow diameter at lower portion 11 and a relatively wider diameter at its upper portion 13. The valve assembly also includes a stem pipe 14 mounted within the bore 12 of the tailpiece and arranged to be axially displaced within the bore 12 against the action of spring 15. The valve stem 14 includes an internal bore 16 having one or more lateral openings (stem holes) 17 (see Figure 2). The valve assembly includes an actuator 18 having a central bore 19 which accommodates the valve stem 14 such that the bore 16 of the stem pipe 14 is in communication with bore 19 of the actuator. A passage 20 in the actuator extending perpendicularly to the bore 19 links the bore 19 with a recess including a post 21 on which is mounted a spraying head in the form of an insert 22 including a bore 23 which is in communication with the passage 20.

A ring 24 of elastomeric material is provided between the outer surface of the valve stem 14 and, ordinarily, this sealing ring closes the lateral opening 17 in the valve stem 14. The construction of the valve assembly is such that when the actuator 18 is manually depressed, it urges the valve stem 14 downwards against the action of the spring 15 as shown in Figure 2 so that the sealing ring 24 no longer closes the lateral opening 17. In this position, a path is provided from the reservoir 2 to the bore 23 of the spraying head so that liquid can be forced, under the pressure of the gas in the can, to the spraying head via a conduit system comprising the dip tube 4, the tailpiece bore 12, the valve stem bore 16, the actuator bore 19 and the passage 20.

An orifice 27 (not shown in Figure 1) is provided in the wall of the tailpiece 8 and constitutes a vapour phase tap whereby the gas pressure in the reservoir 2 can act directly on the liquid flowing through the valve assembly. This increases the turbulence of the liquid. It has been found that an increased charge is provided if the diameter of the orifice 27 is at least 0.76mm.

Preferably the lateral opening 17 linking the valve stem bore 16 to the tailpiece bore 12 is in the form of 2 orifices each having a diameter of not more than 0.51mm to enhance electrostatic charge generation. Further, the diameter of the dip tube 4 is preferably as small as possible, for example, 1.2mm, in order to increase the charge imparted to the liquid. Also, charge generation is enhanced if the diameter of the tailpiece orifice 11 is as small as possible eg not more than about 0.64mm.

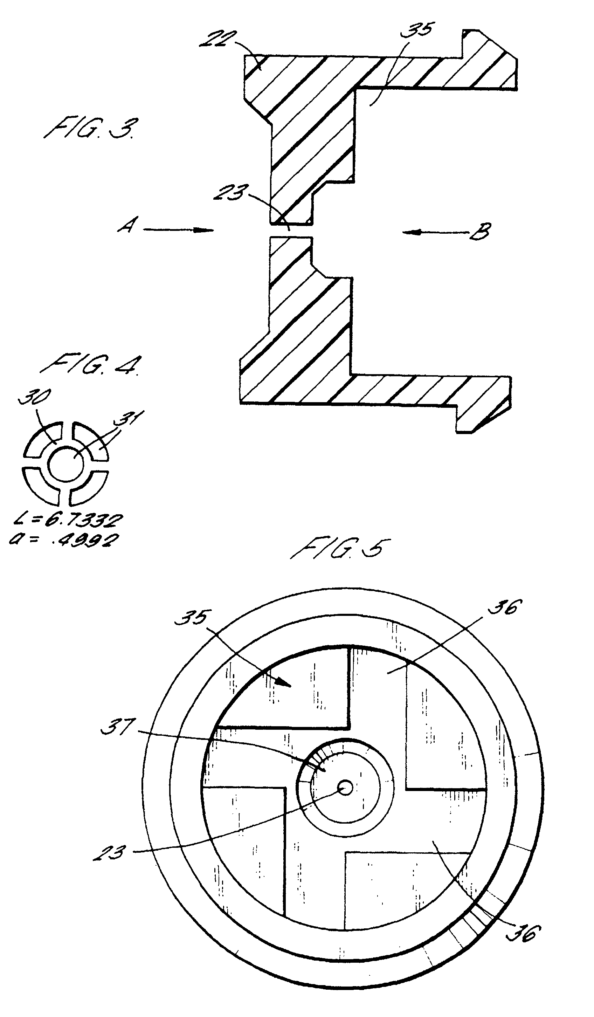

Referring now to Figure 3, there is shown on an increased scale, a cross section through the actuator insert of the apparatus of Figures 1 and 2. For simplicity, the bore 23 is shown as a single cylindrical aperture in this Figure. However, the bore 23 preferably has the configuration, for instance, shown in Figure 4. The apertures of the bore 23 are denoted by reference numeral 31 and the aperture-defining portions of the bore are denoted by reference numeral 30. The total peripheral length of the aperture-defining portions at the bore outlet is denoted by L (in mm) and a is the total area of the aperture at the bore outlet (in mm²) and the values for L and a are as indicated in Figure 4. L/a exceeds 8 and this condition has been found to be particularly conductive to charge development because it signifies, an increased contact area between the actuator insert. and the liquid passing there through.

Many different configurations can be adopted in order to produce a high L/a ratio without the cross-sectional area a being reduced to a value which would allow only low liquid flow rates. Thus, for example it is possible to use actuator insert bore configurations (i) wherein the bore outlet comprises a plurality of segment-like apertures (with or without a central aperture); (ii) wherein the outlet comprises a plurality of sector-like apertures; (iii) wherein the aperture together form an outlet in the form of a grill or grid; (iv) wherein the outlet is generally cruciform; (v) wherein the apertures together define an outlet in the form of concentric rings; and combinations of these configurations. Particularly preferred are actuator insert bore configurations wherein a tongue like portion protrudes into the liquid flow stream and can be vibrated thereby. This vibrational property may cause turbulent flow and enhanced electrostatic charge separation of the double layer, allowing more charge to move into the bulk of the liquid.

Referring now to Figure 5, there is shown a plan view of one possible configuration of swirl chamber 35 of the actuator insert 22. The swirl chamber includes 4 lateral channels 36 equally spaced and tangential to a central area 37 surrounding the bore 23. In use, the liquid driven from the reservoir 2 by the gas under pressure travels along passage 20 and strikes the channels 36 normal to the longitudinal axis of the channels. The arrangement of the channels is such that the liquid tends to follow a circular motion prior to entering the central area 37 and thence the bore 23. As a consequence, the liquid is subjected to substantial turbulence which enhances the electrostatic charge in the liquid.

The following comparative Examples illustrate the principles of the invention::-

### COMPARATIVE EXAMPLE 1

An aerosol disinfectant composition was prepared from the following components:

| | **%w/w** |
|---|---|
| Ethanol | 54 |
| Silicone surfactant | 0.1 |
| Anti-bacterial agent chosen from below | 0.8 |
| Water | 17.2 |
| Liquified petroleum gas | 28 |

The composition was introduced into a tinplate aerosol can having valve assemblies comprising a 3mm polyethylene dip tube 4, 0.64mm tailpiece orifice 11, 0.64mm vapour phase tap 27 and 4 x 0.61mm valve stem lateral openings 17. The actuator 18 was an Kosmos type fitted with a 0.51/0.66mm Aqua actuator insert 22 (both supplied by Precision Valve).

The anti-bacterial agent may be any suitable material. By way of example, an essential oil may be used, including one or more of the following:

Lemon grass, lemon, orange, yeast, clove, thyme, oregano, cinnamaldehye, cinnamon and/or carvacrol.

A preferred amount of the anti-bacterial agent in the composition is from 0.2 to 0.25% w/w.

In order to demonstrate the principle of the invention, the charge level on the droplets emitted from this can was artificially raised to a charge to mass ratio of approximately -1 x 10⁻⁴ C/kg by applying a -10 kv charge to the seam of the can from a high voltage power supply.

On depression of the actuator 18, a fine spray of liquid droplets having a charge/mass ratio of -1 x 10⁻⁴ C/kg and a flow rate of approximately 1.2 g/sec was obtained. The droplets became rapidly dispersed in the air.

The above-described aerosol spray device was compared with a standard, known aerosol spray device loaded with the same aerosol formulation. The following protocol was used.

A suspension of micrococcus luteus containing approximately 10⁹ cfu/ml in water is prepared. HEPA filtered air is supplied to an environmental test chamber with a volume of 28 cubic metres.

The bacterial suspension is applied to the test chamber with a nebuliser for 60 seconds and is distributed around the chamber for a further 60 seconds with a re-circulating fan.

A slit-to-agar sampler is activated for 2 hours obtaining samples after 1, 15, 30, 60 and 120 minutes. The slit-to-agar plates are collected. The plates are assayed, incubated and the colonies counted to provide the control results (which are the average of three experiments).

The above procedure is repeated 3 times, however before activating the slit-to-agar sampler the electrostatically charged test product is sprayed into the test chamber for 10 seconds. This is repeated again 3 times with the conventional non-charged test product.

The results obtained from the charged and non-charged spray products are compared (after taking the control results into account) and it is thereby shown that there is a significant increase in the aerial anti-bacterial performance with the electrostatic product.

### COMPARATIVE EXAMPLE 2

The test organism *Micrococcus lutens* (ATCC NCTC 2665) obtained from culture was used as a final suspension containing about 10⁹ cfu/ml in water. The test chamber was of volume 28m³, provided with HEPA filtered air supply and extracted air. Equipment in the chamber was remotely controlled from a control room. Bacteria were sprayed from a collison nebulizer for 60 seconds and mixed with the room air for a further 60 seconds by a fan. Five all glass impingers (AGI) were activated at the following times 1, 14, 29, 59 and 119 minutes after bacterial release. Each impinger collected air from the test chamber for one minute. After the first impinger had finished sampling a test disinfectant spray was released into the chamber from an aerosol can. The test formulation was:-

| **Component** | **%w/w** |
|---|---|
| Butane 40 | 35 |
| Sorbitan Mono-oleate | 1 |
| Lemon Grass Oil | 7 |
| Sodium Nitrite | 0.12 |
| Triethyleneglycol | 2.5 |
| Soft Water | 54.38 |

The spray was released for 10 seconds. The performance of an uncharged aerosol composition was compared with the same aerosol composition with charge applied artificially to the can in order to demonstrate the principle of the invention. The voltage applied to the can was -3kV, this achieved an aerosol with a charge-to-mass ratio of -1.3 X 10⁻⁴ C/Kg. The effect of applying charge to the aerosol on the concentration of airborne microorganisms was recorded by the following AGI's. The number of bacteria collected in the AGI was assessed by removing 0.1ml of the liquid from the AGI and placing it on an agar plate, this was replicated once. The plates were then incubated at 30°C for 72 hours. The colonies were counted and the average of the 2 plates presented in the results given below:

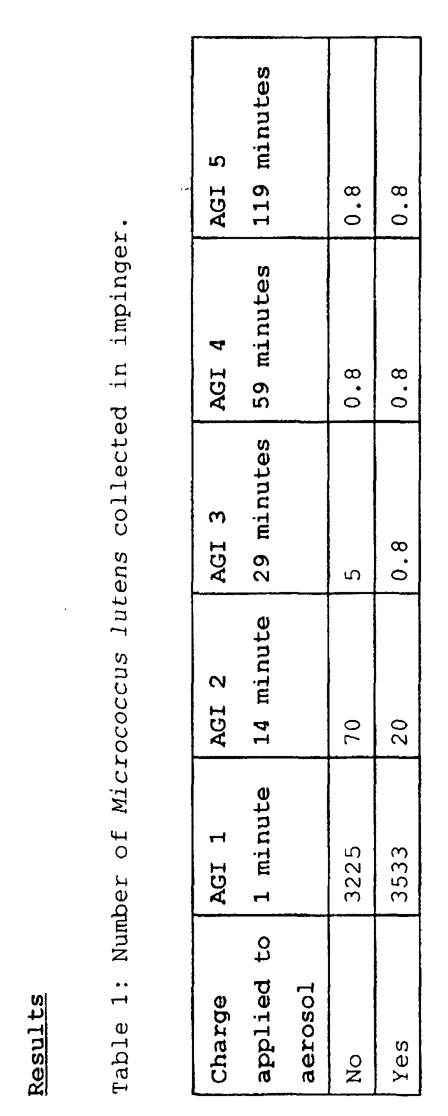

## Claims

1. A method of disinfecting or sanitising a space occupied by airborne microorganisms and/or viruses, which method comprises directing into the space liquid droplets from a spray device containing a disinfecting or sanitising composition, **characterized in that** a unipolar charge is imparted to the said liquid droplets solely by double layer charging during the spraying of the liquid droplets from the spray device, the unipolar charge being at a level such that the said droplets have a charge to mass ratio of at least +/- 1 x 10⁻⁴ C/kg.

2. A method as claimed in claim 1 wherein the spray device is an aerosol spray device.

3. A method as claimed in claim 1 or claim 2 wherein the desinfecting or sanitising composition is an emulsion.

4. A method as claimed in any one of the preceding claims wherein the liquid droplets have a diameter in the range of from 5 to 100 micrometres.

5. A method as claimed in any one of the preceding claims wherein the unipolar charge is imparted to the liquid droplets without any charge being imparted thereto from an internal or external charge inducing device.

6. A method as claimed in claim 5 wherein the droplet charge to mass ratio of at least +/- 1 x 10⁻⁴ C/kg is imparted to the droplets as a result of the use of an aerosol spray device with at least one of the features of the material of the actuator, the size and shape of the orifice of the actuator, the diameter of the dip tube, the characteristics of the valve and the formulation of the disinfecting or sanitising composition contained within the aerosol spray device being chosen in order to achieve the said droplet charge to mass ratio by double layer charging imparting the unipolar charge to the droplets during the actual spraying of the liquid droplets from the orifice of the aerosol spray device.

7. A method as claimed in any one of the preceding claims wherein the disinfecting or sanitising composition comprises an oil phase, an aqueous phase, a surfactant, an anti-bacterial agent, a fungicide or an anti-viral agent, and a propellant.

8. A method as claimed in claim 7 wherein the anti-bacterial or anti-viral agent is an essential oil selected from thyme, lemon grass, lemon, orange, grapefruit, yeast, oregano, anise, clove, cinnamaldehyde, cinnamon, carvacrol, rose, lavender, citronella, eucalyptus, peppermint, camphor, sandalwood, juniper berry, Siberian pine needle, pine sylvester, tea tree, litsea, rosewood, patchouli, vetyver, cedarwood and mixtures thereof.

9. A method as claimed in claim 7 wherein the anti-bacterial agent is a quaternary ammonium compound.

10. A method according to any one of claims 7 to 9 wherein the oil phase includes a C₉ - C₁₂ hydrocarbon.

11. A method as claimed in claim 10 wherein the C₉ -C₁₂ hydrocarbon is present in the composition in an amount of from 2 to 10% w/w.

12. A method as claimed in any one of claims 7 to 11 wherein the surfactant is glyceryl oleate or a polyglycerol oleate.

13. A method as claimed in any one of claims 7 to 12 wherein the surfactant is present in the composition in an amount of from 0.1 to 1.0% w/w.

14. A method as claimed in any one of claims 7 to 13 wherein the propellant is liquified petroleum gas.

15. A method as claimed in claim 14 wherein the propellant is present in the composition in an amount of from 20 to 50% w/w.

## Patentansprüche

1. Verfahren zum Desinfizieren oder Sterilisieren einer durch in der Luft enthaltenen Mikroorganismen und/oder Viren kontaminierten Stelle, wobei das Verfahren das Aufbringen von flüssigen Tröpfchen aus einer Sprayvorrichtung, welche eine Desinfektions- oder Sterilisationszusammensetzung umfasst, auf die Stelle umfasst, **dadurch gekennzeichnet, dass** den flüssigen Tröpfchen nur durch Doppelschichtladung während des Sprayens der flüssigen Tröpfchen aus der Sprayvorrichtung eine unipolare Ladung verliehen wird, wobei die unipolare Ladung in einem Bereich liegt, in dem das Ladungs- zu Masseverhältnis bei mindestens +/- 1 x 10⁻⁴ C/kg liegt.

2. Verfahren gemäß Anspruch 1, wobei die Sprayvorrichtung eine Aerosolsprayvorrichtung ist.

3. Verfahren gemäß Anspruch 1 oder Anspruch 2, wobei die Desinfektions- oder Sterilisationszusammensetzung eine Emulsion ist.

4. Verfahren gemäß einem der vorstehenden Ansprüche, wobei die flüssigen Tröpfchen einen Durchmesser im Bereich von 5 bis 100 µm aufweisen.

5. Verfahren gemäß einem der vorstehenden Ansprüche, wobei den flüssigen Tröpfchen eine unipolare Ladung verliehen wird, ohne dass deren Ladung durch eine interne oder externe ladungsinduzierende Vorrichtung vorgenommen wird.

6. Verfahren gemäß Anspruch 5, wobei den Tröpfchen ein Tröpfchenladungs- zu -masseverhältnis von mindestens +/- 1 x 10⁻⁴ C/kg verliehen wird, indem ein Aerosolspray verwendet wird, bei dem mindestens ein Merkmal aus Sprühkopfmaterial, Größe und Form der Sprühkopföffnung, Durchmesser des Steigrohrs, Eigenschaften des Ventils und der Formulierung der Desinfektions- oder Sterilisationszusammensetzung in der Aerosolsprayvorrichtung so ausgewählt ist, um das Tröpfchenladungs- zu Masseverhältnis durch Doppelschichtladung zu erhalten, wobei den Töpfchen während des tatsächlichen Sprayens der flüssigen Tröpfchen aus der Öffnung der Aerosolsprayvorrichtung eine unipolare Ladung verliehen wird.

7. Verfahren gemäß einem der vorstehenden Ansprüche, wobei die Desinfektions- oder Sterilisationszusammensetzung eine Ölphase, eine wässrige Phase, eine oberflächenaktive Substanz, ein antibakterielles Mittel, ein Fungizid oder ein Anti-Virusmittel und ein Treibmittel umfasst.

8. Verfahren gemäß Anspruch 7, wobei das antibakterielle Mittel oder Anti-Virusmittel ein etherisches Öl umfasst, welches ausgewählt ist aus Thymian, Zitronengras, Zitrone, Orange, Grapefruit, Hefe, Oregano, Anis, Nelke, Zimtaldehyd, Zimt, Carvacrol, Rose, Lavendel, Citronellöl, Eukalyptus, Pfefferminz, Campher, Sandelholz, Wacholderbeere, Sibirische Kiefernnadel, Pinus sylvestris, Teebaum, Litsea cubeba, Rosenholz, Patschuli, Vetyver, Zedernholz und Gemischen davon.

9. Verfahren gemäß Anspruch 7, wobei das antibakterielle Mittel eine quartäre Ammoniumverbindung ist.

10. Verfahren gemäß einem der Ansprüche 7 bis 9, wobei die Ölphase einen C₉-C₁₂-Kohlenwasserstoff einschließt.

11. Verfahren gemäß Anspruch 10, wobei der C₉-C₁₂-Kohlenwasserstoff in einer Menge von 2 bis 10 Gew.-% in der Zusammensetzung vorhanden ist.

12. Verfahren gemäß einem der Ansprüche 7 bis 11, wobei die oberflächenaktive Substanz ein Glyceryloleat oder ein Polyglycerololeat ist.

13. Verfahren gemäß einem der Ansprüche 7 bis 12, wobei die oberflächenaktive Substanz in einer Menge von 0,1 bis 1,0 Gew.-% in der Zusammensetzung vorhanden ist.

14. Verfahren gemäß einem der Ansprüche 7 bis 13, wobei das Treibmittel ein verflüssigtes Petroleumgas ist.

15. Verfahren gemäß Anspruch 14, wobei das Treibmittel in einer Menge von 20 bis 50 Gew.-% in der Zusammensetzung vorhanden ist.

## Revendications

1. Procédé de désinfection ou d'assainissement d'un espace occupé par des micro-organismes et/ou des virus en suspension dans l'air, ce procédé comprenant les étapes consistant à envoyer dans l'espace des gouttelettes liquides, projetées par un dispositif de pulvérisation, contenant une composition de désinfection ou d'assainissement, **caractérisé en ce qu'**une charge unipolaire est appliquée auxdites gouttelettes liquides uniquement par un chargement en double couche pendant la pulvérisation des gouttelettes liquides par le dispositif de pulvérisation, la charge unipolaire étant à un niveau tel que lesdites gouttelettes présentent un rapport charge/masse d'au moins ± 1 x 10⁻⁴ C/kg.

2. Procédé selon la revendication 1, dans lequel le dispositif de pulvérisation est un dispositif de pulvérisation en aérosol.

3. Procédé selon la revendication 1 ou 2, dans lequel la composition de désinfection ou d'assainissement est une émulsion.

4. Procédé selon l'une quelconque des revendications précédentes, dans lequel les gouttelettes liquides ont un diamètre compris entre 5 et 100 micromètres.

5. Procédé selon l'une quelconque des revendications précédentes, dans lequel la charge unipolaire est appliquée aux gouttelettes liquides sans qu'aucune charge ne leur soit appliquée par un dispositif inducteur de charges, interne ou externe.

6. Procédé selon la revendication 5, dans lequel le rapport charge/masse des gouttelettes, qui est d'au moins ± 1 x 10⁻⁴ C/kg, est appliqué aux gouttelettes du fait de l'usage d'un dispositif de pulvérisation en aérosol, au moins une des caractéristiques parmi le matériau de l'actionneur, la taille et la forme de l'orifice de l'actionneur, le diamètre du tube plongeur, les caractéristiques de la vanne et de la formulation de la composition de désinfection ou d'assainissement contenue dans le dispositif de pulvérisation en aérosol étant choisie afin d'obtenir ledit rapport charge/masse des gouttelettes par un chargement en double couche appliquant la charge unipolaire aux gouttelettes pendant la pulvérisation effective des gouttelettes liquides par l'orifice du dispositif de pulvérisation en aérosol.

7. Procédé selon l'une quelconque des revendications précédentes, dans lequel la composition de désinfection ou d'assainissement comprend une phase huileuse, une phase aqueuse, un agent tensio-actif, un agent antibactérien, un fongicide ou un agent antiviral, et un gaz propulseur.

8. Procédé selon la revendication 7, dans lequel l'agent antibactérien ou antiviral est une huile essentielle sélectionnée parmi les suivantes : thym, verveine des Indes, citron, orange, pamplemousse, levure, origan, anis, clou de girofle, cinnamaldéhyde, cannelle, carvacrol, rose, lavande, citronnelle, eucalyptus, menthe poivrée, camphre, bois de santal, baies de genièvre, aiguilles de pin de Sibérie, pin sylvestre, théier, litsée, bois de rose, patchouli, vétiver, bois de cèdre et mélanges de ceux-ci.

9. Procédé selon la revendication 7, dans lequel l'agent antibactérien est un composé d'ammonium quaternaire.

10. Procédé selon l'une quelconque des revendications 7 à 9, dans lequel la phase huileuse comprend un hydrocarbure C₉-C₁₂.

11. Procédé selon la revendication 10, dans lequel l'hydrocarbure C₉-C₁₂ est présent dans la composition dans une quantité de 2 à 10 % en poids par poids.

12. Procédé selon l'une quelconque des revendications 7 à 11, dans lequel l'agent tensio-actif est l'oléate de glycéryle ou l'oléate de polyglycérol.

13. Procédé selon l'une quelconque des revendications 7 à 12, dans lequel l'agent tensio-actif est présent dans la composition dans une quantité de 0,1 à 1,0 % en poids par poids.

14. Procédé selon l'une quelconque des revendications 7 à 13, dans lequel le gaz propulseur est un gaz de pétrole liquéfié.

15. Procédé selon la revendication 14, dans lequel le gaz propulseur est présent dans la composition dans une quantité de 20 à 50 % en poids par poids.
